(19) Europäisches Patentamt
European Patent Office
Office européen des brevets

(11) **EP 4 656 184 A1**

(12) **EUROPEAN PATENT APPLICATION**
published in accordance with Art. 153(4) EPC

(43) Date of publication:
03.12.2025 Bulletin 2025/49

(21) Application number: 24747483.6

(22) Date of filing: 26.01.2024

(51) International Patent Classification (IPC):
*A61K 9/14* (2006.01)    *A61K 47/12* (2006.01)
*A61K 47/18* (2017.01)    *A61K 47/34* (2017.01)
*A61K 47/22* (2006.01)    *A61K 9/00* (2006.01)
*A61K 38/21* (2006.01)    *A61P 25/28* (2006.01)
*A61P 37/00* (2006.01)    *A61P 35/00* (2006.01)

(52) Cooperative Patent Classification (CPC):
A61K 9/00; A61K 9/14; A61K 38/21; A61K 47/12;
A61K 47/18; A61K 47/22; A61K 47/34;
A61P 25/28; A61P 35/00; A61P 37/00

(86) International application number:
PCT/KR2024/001270

(87) International publication number:
WO 2024/158250 (02.08.2024 Gazette 2024/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 26.01.2023 KR 20230010191

(71) Applicant: Abion Inc.
Seoul 08394 (KR)

(72) Inventors:
• CHOI, Hong Seok
  Seoul 08394 (KR)
• KIM, Na Young
  Seoul 08394 (KR)
• CHOI, Jun Young
  Seoul 08394 (KR)

(74) Representative: JWP Patent & Trademark
Attorneys
ul. Minska 75
03-828 Warszawa (PL)

(54) **DRY POWDER FORMULATION OF INTERFERON BETA AND METHOD FOR PREPARING SAME**

(57)    The present invention relates to a dry powder formulation of interferon beta and a method for preparing same and, more specifically, to a dry powder formulation of interferon beta in the form of spherical particles having a high degree of morphological perfection, prepared by spray-drying a liquid composition containing interferon beta, and a method for preparing the dry powder formulation of interferon beta.

Fig. 1

**Description**

**[Technical Field]**

The present application claims priority to Korean Patent Application No. 10-2023-0010191, filed on January 26, 2023, and the entire disclosure of the aforementioned application is incorporated herein by reference.

The present disclosure relates to a dry powder formulation of interferon-beta and a method for preparing the same, and more specifically, the present disclosure relates to a dry powder formulation of interferon-beta comprising spherical particles with high morphological integrity, which is prepared by spray drying a liquid composition comprising interferon-beta, and a method for preparing the same.

**[Background Art]**

Interferons (IFNs) are a type of cytokine that exhibit antiviral activity, inhibit cell proliferation, and regulate innate immune responses. IFNs are classified into IFN-$\alpha$, IFN-$\beta$, and IFN-$\gamma$ depending on their cellular origin (leukocytes, fibroblasts, T cells). Among them, interferon-beta (IFN-$\beta$) is a globular protein with five alpha-helices ($\alpha$-helix), has a size of 22 kD, and becomes 18 kD when the glycan is removed.

Research on the clinical application of IFN-$\beta$ is actively ongoing, and it is particularly gaining attention as a therapeutic agent for alleviating, mitigating, or treating symptoms of multiple sclerosis (MS). Additionally, it has been reported to be effective in treating various conditions such as cancer, autoimmune disorders, viral infections, HIV-related diseases, hepatitis C, and rheumatoid arthritis due to its diverse immunological activities, including antiviral activity, inhibition of cell growth, enhancement of lymphocyte cytotoxicity, immunomodulatory activity, induction or inhibition of target cell differentiation, macrophage activation, increased cytokine production, enhancement of cytotoxic T-cell efficacy, and increased activity of natural killer cells.

Human IFN-$\beta$ is also a type of glycoprotein, and the glycan portion attached to the protein plays an important role in the protein's activity. In the case of glycoproteins, the addition of glycans can sometimes increase their activity. Specifically, it is known that protein glycosylation can affect many biochemical properties such as stability, solubility, intracellular trafficking activity, pharmacokinetics, and antigenicity.

IFNs are generally formulated as isotonic solutions for parenteral administration. Recently, clinicians have been seeking alternative routes of IFN administration that are more suitable for long-term use in patients. Specifically, aerosol formulations of IFN have been developed for pulmonary delivery, as described in WO91/16038. These formulations are dispersed by the vaporization of liquid propellants. The patent describes improving the dispersibility of human IFN from a Freon delivery system by adding surfactants or their analogs.

Methods and compositions for preparing solid polypeptide microparticles as pharmaceutical aerosol formulations are described in WO91/16038, where IFN-$\beta$ was prepared in dry powder form by freeze-drying an IFN solution and then jet-milling the freeze-dried product.

However, such dry powder forms had the issue of not exhibiting a completely spherical particle shape, making them unsuitable for effective pulmonary delivery. Additionally, compared to the liquid formulation prior to the drying process, there was a limitation that the activity of IFN was significantly reduced.

**[Technical Problem]**

Accordingly, the present inventors have conducted repeated studies to develop a dry powder formulation with high morphological completeness and interferon activity. As a result, the present inventors confirmed that spray drying a liquid interferon beta composition comprising acetate buffer, arginine, poloxamer 188, methionine, and hydrophobic amino acids can produce a dry powder formulation of interferon-beta with excellent morphological completeness and activity, thereby completing the present disclosure.

Therefore, an object of the present disclosure is to provide a dry powder formulation of interferon-beta prepared by spray drying a liquid composition, wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

Another object of the present disclosure is to provide an inhalation capsule filled with the dry powder formulation.
Another object of the present disclosure is to provide a pharmaceutical composition for preventing or treating a disease selected from the group consisting of multiple sclerosis, cancer, autoimmune diseases, viral infectious diseases, HIV infectious diseases, hepatitis C, and rheumatoid arthritis, comprising the dry powder formulation.
Additionally, another object of the present disclosure is to provide a pharmaceutical composition for preventing or treating a disease selected from the group consisting of multiple sclerosis, cancer, autoimmune diseases, viral infectious diseases, HIV infectious diseases, hepatitis C, and rheumatoid arthritis, consisting of the dry powder formulation.
Furthermore, another object of the present disclosure is to provide a pharmaceutical composition for preventing or treating a disease selected from the group consisting of multiple sclerosis, cancer, autoimmune diseases, viral infectious diseases, HIV infectious diseases, hepatitis C, and rheumatoid arthritis, consisting essentially of the dry powder formulation.
Another object of the present disclosure is to provide a method for preparing an dry powder formulation of interferon beta, comprising spray drying a liquid composition at an inlet temperature of 90 to 130°C and an outlet temperature of 40 to 80°C, wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

[Technical Solution]

To achieve the aforementioned objectives of the present disclosure, the present disclosure provides a dry powder formulation of interferon-beta prepared by spray drying a liquid composition, wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

To achieve another objective of the present disclosure, the present disclosure provides an inhalation capsule filled with the dry powder formulation.
To achieve another objective of the present disclosure, the present disclosure provides a pharmaceutical composition for preventing or treating a disease selected from the group consisting of multiple sclerosis, cancer, autoimmune diseases, viral infectious diseases, HIV infectious diseases, hepatitis C, and rheumatoid arthritis, comprising the dry powder formulation.
Additionally, the present disclosure provides a pharmaceutical composition for use in preventing or treating a disease selected from the group consisting of multiple sclerosis, cancer, autoimmune diseases, viral infectious diseases, HIV infectious diseases, hepatitis C, and rheumatoid arthritis, consisting of the dry powder formulation.
Furthermore, the present disclosure provides a pharmaceutical composition for use in preventing or treating a disease selected from the group consisting of multiple sclerosis, cancer, autoimmune diseases, viral infectious diseases, HIV infectious diseases, hepatitis C, and rheumatoid arthritis, consisting essentially of the dry powder formulation.

To achieve another objective of the present disclosure, the present disclosure provides a method for preparing an dry powder formulation of interferon beta, comprising spray drying a liquid composition at an inlet temperature of 90 to 130°C and an outlet temperature of 40 to 80°C, wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

Hereinafter, the present disclosure will be described in detail.

The present disclosure provides a dry powder formulation of interferon-beta prepared by spray drying a liquid composition wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

One of the most fundamental issues in preparing protein-based formulations is achieving the desired therapeutic protein concentration in the solution, as protein stability depends not only on protein concentration but also on the pH, temperature, ionic strength, and additive concentration of the solution. Therefore, protein-based formulations must be suitable for stabilizing therapeutic proteins and preventing issues such as aggregation, precipitation, or fragmentation. When preparing protein-based dry powder formulations, since the proteins dispersed in the solution are powderized through methods such as freeze-drying or spray drying, it is essential to have a composition in which the proteins remain stably dispersed in the solution.

Furthermore, when protein-based dry powder formulations are used for inhalation administration, it is desirable to prepare a dry powder formulation with morphologically high integrity, that is, particles with morphological characteristics close to a spherical shape, so that they can be uniformly absorbed in the nasal mucosa or lungs.

Interferon refers to a type of low molecular weight protein and glycoprotein (sometimes referred to as cytokines) with a molecular weight of approximately 15,000 to 27,000 daltons, which are naturally or recombinantly produced and exhibit interferon activity. Generally, this activity is exerted by binding to specific membrane receptors on the cell surface. Once bound, interferon initiates a complex series of intracellular processes that vary depending on the type of interferon. Interferons are useful for treating various human diseases ranging from cancer to immune system suppression. Natural interferons are produced and secreted by cells in response to viral infections or to synthetic and biological inducers. Some interferons are modified forms of natural substances and are prepared using recombinant DNA technology. Interferons are sometimes abbreviated as "IFN," and this abbreviation will also be used herein. Examples of interferons include IFN-alpha-2A recombinant (RoferonR A-Roche Laboratories), IFN-alpha-2B recombinant (IntronR A-Shering), IFN-alpha-N3 derived from human leukocytes (AlferonR N-Purdue Frederick), IFN-gamma-1B (ActimmuneR-Genentech), IFN-beta recombinant (BetaseronR-Chiron, Berlex), and natural IFN-beta (FeronR-Toray, Japan). As used herein, the term "interferon-beta (IFN-$\beta$)" refers to fibroblast interferon of human origin obtained by isolation from biological fluids or by DNA recombinant technology from eukaryotic or prokaryotic host cells, as well as its salts, functional derivatives, variants, analogs, and active fractions.

Preferably, the IFN-β of the present disclosure represents human IFN-β.

The preferred IFN-β may mean IFN-β-1a and may comprise N-linked glycans at one or more asparagine residues of the protein's amino acid residues.

In a preferred embodiment of the present disclosure, the IFN-β variant may be a variant in which the 27th amino acid, arginine, of human IFN-β is substituted with threonine (R27T). The R27T variant is a recombinant human IFN-β variant (hereinafter referred to as rhINF-β) designed by substituting threonine (Thr) for arginine (Arg) at the 27th position for additional glycosylation at the 25th position of IFN-β 1a, and glycans may be bound at the 25th and 80th amino acids.

In the present disclosure, the term "liquid composition" means that the components (a) to (g) are comprised in a solvent. The type of solvent is not particularly limited as long as it is pharmaceutically acceptable, and preferably, it is water.

In the present disclosure, the term "powder" refers to a formulation consisting of finely dispersed solid particles that are free-flowing, can be easily dispersed by an inhalation device, and subsequently inhaled by a patient, with the particles reaching and penetrating the nasal mucosa or lungs. Therefore, the powder is referred to as "respirable." The average size of the particles is preferably less than about 10 microns ($\mu$m) in diameter, with a relatively uniform elliptical distribution. More preferably, the diameter is less than about 7.5 $\mu$m, and most preferably, less than about 5.0 $\mu$m. The particle size distribution is generally about 0.1 $\mu$m to about 5 $\mu$m in diameter, particularly about 2 $\mu$m to about 5 $\mu$m.

In the present disclosure, the term "dry" means that the composition has a moisture content that allows the particles to be easily dispersed in an inhalation device to form an aerosol. The moisture content is generally less than about 10% by weight of water, usually less than about 5% by weight, and preferably less than about 4% by weight.

According to one embodiment of the present disclosure, the dry powder formulation of IFN-β prepared by spray drying a liquid composition, which comprises (a) interferon beta or a variant thereof; (b) an acetate buffer at a concentration of 5 to 100 mM; (c) arginine at a concentration of 10 to 150 mM; (d) poloxamer 188 at a concentration of 0.1 to 10 mg/mL; (e) methionine at a concentration of 0.5 to 5 mM; and (f) 5 to 30% (w/v) of a hydrophobic amino acid, exhibits a highly morphologically spherical shape with integrity, high recovery rate, and stable activity.

This is, arginine, poloxamer 188, and methionine not only exert an effect to maintain the protein stability of IFN-β at a high level in the liquid composition for spray drying, but also allow the protein to be uniformly dispersed without being destroyed during the spray drying process, thereby enabling the production of particles that morphologically exhibit a shape close to a sphere.

In the present disclosure, the liquid composition for spray drying may comprise arginine at a concentration of 10 to 150 mM, poloxamer 188 at a concentration of 0.1 to 10 mg/mL, and methionine at a concentration of 0.5 to 5 mM, and more preferably, arginine at a concentration of 50 to 100 mM, poloxamer 188 at a concentration of 0.1 to 1 mg/mL, and methionine at a concentration of 0.5 to 2 mM.

If the concentrations of arginine, poloxamer 188, and methionine deviate from the above ranges, the particle size and shape after spray drying may not be uniform, and the pharmacokinetics as an inhalation formulation may not be desirable.

In the present disclosure, the acetate buffer is a solution that has the effect of adjusting or maintaining the pH of the formulation to fall within a desirable pH range for the formulation, and preferably has a concentration of 5 to 100 mM, more preferably a concentration of 10 to 30 mM.

In the present disclosure, the desirable pH range of the acetate buffer may be in the range of 3.6 to 4.4, which is a pH that maintains the stability of interferon beta in the liquid composition for spray drying, prevents abnormal aggregation reactions, and maintains protein stability during the spray drying process while allowing the spherical shape of the powder particles to be well-formed.

According to one embodiment of the present disclosure, when the dry powder prepared by spray drying the IFN-β liquid composition comprising the component according to the present disclosure was rehydrated and the content of IFN-β in the dry powder was examined, it was confirmed that 100% of the IFN-β before spray drying was recovered.

The therapeutically effective amount of IFN-β may vary within the formulation depending on the biological activity of the IFN-β used and the amount required for the unit dosage form. Since IFN-β has high activity, it should be prepared in unit doses to be easily handled by formulation apparatus and consumers. This generally means that the unit dose is about 0.5 mg to 15 mg, preferably about 2 mg to 10 mg, of the total components in the dry powder formulation. Preferably, the amount of IFN-β in the dry powder formulation may vary from about 0.05% (w/w) to about 5.0% (w/w).

To adjust the amount of IFN-β in the dry powder to the above level, IFN-β in the liquid composition may be comprised at a concentration of 0.01 to 1% (w/v), but is not limited thereto.

The dry powder formulation according to the present disclosure may be spray-dried and powdered together with a pharmaceutically acceptable carrier. The amount of the pharmaceutically acceptable carrier is the amount required to provide stability, dispersibility, consistency, and filling properties required to uniformly ensure pulmonary delivery of the composition to the patient in need. Numerically, the amount may be about 95.0% (w/w) to about 99.95% (w/w) depending on the activity of the IFN-β used. It may be preferable to use about 98% (w/w) to about 99.8% (w/w).

The carrier may be composed of a combination of one or more pharmaceutically acceptable excipients, but it is generally free of "penetration enhancers." "Penetration enhancers" are surfactant compounds that promote the penetration of drugs through the mucosa or mucosal wall and are proposed for use in nasal, rectal, and vaginal drug formulations. Penetration enhancers comprise, for example, bile salts such as taurocholate, glycocholate, and deoxycholate, fusidates such as taurodihydrofusidate, and biologically acceptable surfactants such as Tweens and laureth-9. However, the use of penetration enhancers in formulations for the lungs is generally undesirable because the epithelial blood barrier of the lungs may be adversely affected by the surfactant compounds. The dry powder composition of the present disclosure is easily absorbed into the lungs without the need for penetration enhancers.

In the present disclosure, the types of pharmaceutically acceptable excipients useful as carriers comprise stabilizers such as human serum albumin (HSA), bulking agents such as carbohydrates, amino acids, and polypeptides, pH adjusters or buffers, salts such as sodium chloride, and other excipients. The carrier may be crystalline or amorphous form, or a mixture thereof.

Particularly useful bulking agents may comprise acceptable carbohydrates, polypeptides, amino acids, or combinations thereof.

The hydrophobic amino acid in the composition of the present disclosure may be comprised to improve the stereomorphic stability during spray drying and to enhance the dispersibility of the powder. These hydrophobic amino acids may be selected from the group consisting of glycine, alanine, valine, leucine, isoleucine, proline, phenylalanine, and tryptophan, and preferably leucine.

In one aspect of the present disclosure, the hydrophobic amino acid may be comprised in the liquid composition at 15 to 25% (w/v), preferably 17 to 23% (w/v), and most preferably 19 to 21% (w/v).

In one aspect of the present disclosure, the hydrophobic amino acid may be comprised in the liquid composition at 15% (w/v), 16% (w/v), 17% (w/v), 18% (w/v), 19% (w/v), 20% (w/v), 21% (w/v), 22% (w/v), 23% (w/v), 24% (w/v), or 25% (w/v).

If the concentration of the hydrophobic amino acid in the liquid composition is less than 15% (w/v), the yield of the dry powder formulation may decrease, and if it exceeds 25% (w/v), the morphological characteristics of the dry powder particles may not be desirable.

In another aspect of the present disclosure, the hydrophobic amino acid may be leucine and may be comprised in the liquid composition at 15 to 25% (w/v), preferably 17 to 23% (w/v), and most preferably 19 to 21% (w/v).

In another aspect of the present disclosure, the leucine may be comprised in the liquid composition at 15% (w/v), 16% (w/v), 17% (w/v), 18% (w/v), 19% (w/v), 20% (w/v), 21% (w/v), 22% (w/v), 23% (w/v), 24% (w/v), or 25% (w/v).

If the concentration of leucine in the liquid composition is less than 15% (w/v), the yield of the dry powder formulation may decrease, and if it exceeds 25% (w/v), the morphological characteristics of the dry powder particles may not be desirable.

In one aspect of the present disclosure, the formulation may be characterized in that 90% (w/w) of the dry powder has a particle size of $0.1\ \mu$m to $10\ \mu$m, preferably a particle size of $1\ \mu$m to $10\ \mu$m, more preferably a particle size of $3\ \mu$m to $8\ \mu$m, and most preferably a particle size of $5\ \mu$m to $7\ \mu$m.

In one aspect of the present disclosure, the dry powder formulation may be for inhalation administration.

The term "inhalation administration" means administration through the oral or nasal cavity due to the structure of the respiratory system, for example, by spraying or injecting a carrier containing IFN-β or IFN-β in a liquid, aerosol, or gaseous state through the oral or nasal cavity to contact respiratory cells.

In the present disclosure, the term "respiratory system" refers to the organs involved in respiration as a whole, comprising each organ from the nose and mouth to the trachea and lungs. Preferably, in the present disclosure, the respiratory system comprises the nasal mucosa, nasopharynx, oropharynx, laryngopharynx, larynx, trachea, bronchi, bronchioles, and lungs.

The present disclosure also provides an inhalation capsule filled with the dry powder formulation according to the present disclosure.

The inhalation capsule according to the present disclosure may be capsules or cartridges (such as gelatin or hypromellose), or blisters (such as layered aluminum films), which are used in inhalation devices, and are filled with the dry powder formulation according to the present disclosure.

The capsule may have various internal capacities depending on the size, for example, size 0 capsules may have an internal capacity of about 0.68 mL, size 1 capsules about 0.47 mL, size 2 capsules about 0.37 mL, size 3 capsules about 0.27 mL, and size 4 capsules about 0.20 mL. The size of the capsule may be appropriately selected by a person skilled in the art and prepared as a capsule.

The capsule may be transparent in that the patient can visually confirm whether the dry powder formulation inside the capsule has been inhaled after inhalation. In addition, the transparent capsule allows visual confirmation of degradation in stability or product defects such as aggregation or discoloration of the dry powder formulation contained inside.

The inhalation capsule may be administered using any known dry powder inhalation device. The dry powder inhalation device may comprise means for rupturing the capsule, perforating the capsule, or otherwise opening the capsule to deliver the active ingredient in the metered capsule to the patient's lungs. Additionally, it may further comprise an air inlet for forming an airflow, an outlet through which the active ingredient is discharged by the patient inhaling with their mouth, and a sieve for filtering foreign substances. Examples of such devices include commercially available ROTAHALER® by GlaxoSmithKline, HANDIHALER® by Boehringer Ingelheim, or AEROLIZER® by Plastiape, but are not limited thereto.

In the present disclosure, the inhalation capsule may be for the prevention or treatment of a disease selected from the group consisting of multiple sclerosis, cancer, autoimmune diseases, viral infectious diseases, HIV infectious diseases, hepatitis C, and rheumatoid arthritis, but is not limited thereto.

The present disclosure also provides a pharmaceutical composition for the prevention or treatment of a disease selected from the group consisting of multiple sclerosis, cancer, autoimmune diseases, viral infectious diseases, HIV infectious diseases, hepatitis C, and rheumatoid arthritis, comprising the dry powder formulation.

In one aspect of the present disclosure, the viral infectious disease may be a respiratory viral infectious disease. The respiratory virus may be selected from the group consisting of adenovirus, avian influenza virus, bocavirus, coronavirus, cytomegalovirus, hantavirus, herpes simplex virus, influenza virus, measles virus, metapneumovirus, parainfluenza virus, respiratory syncytial virus, rhinovirus, and varicella-zoster virus. Preferably, in the present disclosure, the respiratory virus is a coronavirus.

In the present disclosure, the coronavirus may be: i) an alpha-coronavirus selected from 229E, NL63 infecting humans, or porcine epidemic diarrhea virus (PEDV), (porcine) transmissible gastroenteritis virus (TGEV), canine coronavirus (CCoV), feline coronavirus (FCoV), Miniopterus bat coronavirus 1, Miniopterus bat coronavirus HKU8, Rhinolophus bat coronavirus HKU2, and Scotophilus bat coronavirus 512 not infecting humans; ii) a beta-coronavirus selected from OC43, HKU1, SARS-CoV, MERS-CoV, SARS-CoV-2 infecting humans, or porcine hemagglutinating encephalomyelitis virus (PHEV), bovine coronavirus (BCoV), equine coronavirus (EqCoV), murine coronavirus (MuCoV), Tylonycteris bat coronavirus HKU4, Pipistrellus bat coronavirus HKU5, and Rousettus bat coronavirus HKU9 not infecting humans; iii) a gamma-coronavirus selected from avian coronavirus and Beluga whale coronavirus SW1 not infecting humans; or iv) a delta-coronavirus selected from Bulbul coronavirus HKU11, Thrush coronavirus HKU12, and Munia coronavirus HKU13 not infecting humans.

The pharmaceutical composition of the present disclosure may further comprise a pharmaceutically acceptable carrier.

The pharmaceutically acceptable carrier may further comprise, for example, carriers for oral administration or carriers for parenteral administration. Carriers for oral administration may comprise lactose, starch, cellulose derivatives, magnesium stearate, stearic acid, and the like. Additionally, carriers for parenteral administration may comprise water, suitable oils, saline, aqueous glucose, glycols, and the like. Furthermore, stabilizers and preservatives may be additionally included. Suitable stabilizers comprise antioxidants such as sodium bisulfite, sodium sulfite, or ascorbic acid. Suitable preservatives comprise benzalkonium chloride, methyl- or propyl-paraben, and chlorobutanol. Other pharmaceutically acceptable carriers may be referred to as known in the art.

The pharmaceutical composition of the present disclosure may be administered to mammals, including humans, by any method. For example, it may be administered orally or parenterally, and parenteral administration methods may comprise, but are not limited to, intravenous, intramuscular, intra-arterial, intramedullary, intradural, intracardiac, transdermal, subcutaneous, intraperitoneal, intranasal, enteral, topical, sublingual, or rectal administration, and preferably intranasal administration.

The pharmaceutical composition of the present disclosure may be formulated as a preparation for oral or parenteral administration according to the above-described administration routes. When formulating, it may be prepared using one or more buffering agents (e.g., saline or PBS), antioxidants, bacteriostatic agents, chelating agents (e.g., EDTA or glutathione), fillers, extenders, binders, adjuvants (e.g., aluminum hydroxide), suspending agents, thickening agents, wetting agents, disintegrants, surfactants, diluents, or excipients.

Solid preparations for oral administration comprise tablets, pills, powders, granules, liquids, gels, syrups, slurries, suspensions, capsules, or other solid preparations. And such solid preparations may be prepared by mixing the pharmaceutical composition of the present disclosure with at least one excipient, for example, starch (including corn starch, wheat starch, rice starch, potato starch, etc.), calcium carbonate, sucrose, lactose, dextrose, sorbitol, mannitol, xylitol, erythritol maltitol, cellulose, methyl cellulose, sodium carboxymethyl cellulose, hydroxypropyl methylcellulose, or gelatin. For example, tablets or sugar-coated tablets may be obtained by mixing the active ingredient with a solid excipient, grinding the mixture, adding a suitable auxiliary agent, and processing it into a granulated mixture.

In addition to simple excipients, lubricants such as magnesium stearate and talc are also used. Liquid preparations for oral administration comprise suspensions, solutions, emulsions, or syrups, and may comprise various excipients, for example, wetting agents, sweeteners, flavoring agents, or preservatives, in addition to simple diluents such as water or liquid paraffin.

Additionally, disintegrants such as cross-linked polyvinylpyrrolidone, agar, alginic acid, or sodium alginate may be added as needed, and anti-caking agents, lubricants, wetting agents, flavoring agents, emulsifiers, and preservatives may also be included.

When administered parenterally, the pharmaceutical composition of the present disclosure can be formulated in the form of an injection, transdermal preparation, or nasal inhalant in accordance with methods known in the art, together with a suitable parenteral carrier. In the case of injections, they must be sterile and protected from contamination by microorganisms such as bacteria and fungi. Examples of suitable carriers for injections comprise, but are not limited to, solvents or dispersion media comprising water, ethanol, polyols (e.g., glycerol, propylene glycol, and liquid polyethylene glycol), mixtures thereof, and/or vegetable oils. More preferably, suitable carriers may comprise isotonic solutions such as Hank's solution, Ringer's solution, phosphate-buffered saline (PBS) containing triethanolamine, sterile water for injection, 10% ethanol, 40% propylene glycol, and 5% dextrose. To protect the injection from microbial contamination, various antimicrobial and antifungal agents such as parabens, chlorobutanol, phenol, sorbic acid, and thimerosal may be additionally included. Furthermore, the injection may, in most cases, additionally include isotonic agents such as sugars or sodium chloride.

For transdermal preparations, forms such as ointments, creams, lotions, gels, external solutions, pastes, liniments, and aerosols are included. Here, "transdermal administration" refers to the topical application of the pharmaceutical composition to the skin, allowing an effective amount of the active ingredient contained in the pharmaceutical composition to be delivered into the skin.

For inhalation preparations, the composition according to the present disclosure can be conveniently delivered in the form of an aerosol spray from a pressurized pack or nebulizer using a suitable propellant, such as dichlorofluoromethane, trichlorofluoromethane, dichlorotetrafluoroethane, carbon dioxide, or other suitable gases. In the case of pressurized aerosols, the dosage unit can be determined by providing a valve that delivers a metered amount. For example, gelatin capsules and cartridges used in inhalers or insufflators can be formulated to contain a powder mixture of the compound and a suitable powder base such as lactose or starch. Formulations for parenteral administration are described in guidelines generally known in pharmaceutical chemistry.

The pharmaceutical composition of the present disclosure can provide desirable effects for the prevention and treatment of diseases when it comprises IFN-β in an effective amount. As used herein, the term "effective amount" refers to an amount that exhibits a response greater than that of a negative control group and preferably an amount sufficient to alleviate the aforementioned pathological symptoms. The pharmaceutical composition of the present disclosure may comprise IFN-β in an amount of 0.01 to 99.99%, with the remainder being a pharmaceutically acceptable carrier. The effective amount of IFN-β comprised in the pharmaceutical composition of the present disclosure may vary depending on the form in which the composition is commercialized.

The total effective amount of the pharmaceutical composition of the present disclosure can be administered to a patient as a single dose or by a fractionated treatment protocol involving multiple doses over an extended period. The pharmaceutical composition of the present disclosure may vary the content of the active ingredient depending on the severity of the disease. For parenteral administration, IFN-β may preferably be administered in an amount of 0.01 to 50 mg, more preferably 0.1 to 30 mg, per kilogram of body weight per day. For oral administration, IFN-β may preferably be administered in an amount of 0.01 to 100 mg, more preferably 0.01 to 10 mg, per kilogram of body weight per day, divided into one or several doses. However, the dosage of IFN-β is determined as an effective dose for the patient, taking into account various factors such as the route of administration, frequency of treatment, age, weight, health condition, gender, severity of the disease, diet, and excretion rate of the patient. Considering these factors, one skilled in the art can determine an appropriate effective dose of IFN-β for the specific use of preventing and treating the aforementioned diseases. The pharmaceutical composition according to the present disclosure is not particularly limited in its formulation, route of administration, or method of administration, as long as it exhibits the effects of the present disclosure.

The pharmaceutical composition of the present disclosure can also be provided in the form of a topical formulation containing IFN-β as an active ingredient.

When the pharmaceutical composition of the present disclosure is used as a topical preparation for the skin, it may additionally include auxiliary agents commonly used in the field of dermatology, such as lipids, organic solvents, solubilizers, thickeners and gelling agents, emollients, antioxidants, suspending agents, stabilizers, foaming agents, fragrances, surfactants, water, ionic emulsifiers, nonionic emulsifiers, fillers, metal ion sequestrants, chelating agents, preservatives, vitamins, blockers, humectants, essential oils, dyes, pigments, hydrophilic activators, lipophilic activators, lipid vesicles, or any other components generally used as a topical preparation for the skin. These components may also be introduced in amounts generally used in the field of dermatology.

When the pharmaceutical composition of the present disclosure is provided as a topical preparation for the skin, it may be in the form of, but not limited to, ointments, patches, gels, creams, or sprays.

The present disclosure also provides a method for preparing a dry powder formulation of interferon beta, comprising spray drying a liquid composition at an inlet temperature of 90 to 130°C and an outlet temperature of 40 to 80°C, wherein the liquid composition comprises:

(a) interferon beta or a variant thereof;

(b) an acetate buffer at a concentration of 5 to 100 mM;

(c) arginine at a concentration of 10 to 150 mM;

(d) poloxamer 188 at a concentration of 0.1 to 10 mg/mL;

(e) methionine at a concentration of 0.5 to 5 mM; and

(f) 5 to 30% (w/v) of a hydrophobic amino acid.

The method for preparing, the inlet temperature may preferably be 100 to 120°C, and the outlet temperature may preferably be 60 to 70°C.

The present disclosure also provides the use of the above dry powder formulation for preparing a composition for treating a disease selected from the group consisting of multiple sclerosis, cancer, autoimmune diseases, viral infectious diseases, HIV infectious diseases, hepatitis C, and rheumatoid arthritis.

The present disclosure also provides a method for treating a disease selected from the group consisting of multiple sclerosis, cancer, autoimmune diseases, viral infectious diseases, HIV infectious diseases, hepatitis C, and rheumatoid arthritis, comprising administering an effective amount of a composition comprising the above dry powder formulation as an active ingredient to a subject in need thereof.

As used herein, the term "effective amount" refers to an amount that, when administered to a subject, exhibits an effect of improving, treating, detecting, diagnosing, or suppressing or reducing a disease selected from the group consisting of multiple sclerosis, cancer, autoimmune diseases, viral infectious diseases, HIV infectious diseases, hepatitis C, and rheumatoid arthritis. The term "subject" may comprise animals, preferably mammals, particularly humans, and may also comprise cells, tissues, or organs derived from animals. The subject may be a patient in need of the above effect.

As used herein, the term "treatment" as used in the present disclosure comprehensively refers to improving a disease selected from the group consisting of multiple sclerosis, cancer, autoimmune diseases, viral infectious diseases, HIV infectious diseases, hepatitis C, and rheumatoid arthritis, or improving symptoms caused by the disease. This may comprise curing, substantially preventing, or improving the condition, as well as alleviating, curing, or preventing one or most symptoms caused by the disease, but is not limited thereto.

As used herein, the term "comprising" as used herein is employed in the same sense as "including" or "characterized by," and does not exclude additional components or steps of methods that are not specifically mentioned in the composition or method according to the present disclosure. Furthermore, the term "consisting of" means excluding additional elements, steps, or components that are not separately described. The term "consisting essentially of" means that, within the scope of the composition or method, it may include substances or steps that do not substantially affect the fundamental characteristics of the described substances or steps.

**[Advantageous Effects]**

The dry powder formulation of IFN-β according to the present disclosure is a dry powder formulation of IFN-β with excellent morphological completeness and activity, which can effectively deliver IFN-β to the respiratory tract through inhalation administration. Therefore, it can be very usefully utilized for the effective *in vivo* delivery of IFN-β, including the treatment of respiratory diseases and respiratory viral infections.

**[Brief Description of the Drawings]**

Figure 1 is a diagram of dry powder particles under a scanning electron microscope (SEM) after spray drying a liquid composition comprising IFN-β (Batch 1: dry powder formulation of a liquid composition without leucine, Batch 2: dry powder formulation of a liquid composition comprising 10% (w/v) leucine, Batch 3: dry powder formulation of a liquid composition comprising 20% (w/v) leucine).

Figure 2 is the result of an ELISA analysis by rehydrating the dry powder formulation to evaluate the antiviral activity of the dry powder formulation of IFN-β according to the present disclosure. A liquid composition of IFN-β that was not spray-dried was used as a control group.

Figure 3 is the result of a CPE (cytopathic effect) analysis by rehydrating the dry powder formulation to evaluate the antiviral activity of the dry powder formulation of IFN-β according to the present disclosure. A liquid composition of IFN-β that was not spray-dried was used as a control group.

**[Detailed Description of the Invention]**

Hereinafter, the present disclosure will be described in detail with reference to the following examples. However, the following examples are merely illustrative of the present disclosure, and the present disclosure is not limited thereto.

**Example 1: Manufacture and Particle Size Analysis of Dry Powder of Interferon-Beta (Dry-Powder Inhalation (DPI))**

To manufacture the DPI(dry powder formulation of interferon-beta), particle size analysis was performed during the DPI conversion of the liquid composition of interferon-beta.

For the preparation of drying powder particles, an interferon-beta liquid composition of interferon-beta (1 mg/mL human interferon-beta R27T variant, 50 mM arginine, 0.5 mg/mL poloxamer 188, 1 mM methionine, and 10% (w/v) or 20% (w/v) leucine in 20 mM acetate buffer at pH 3.8) was spray-dried under the conditions shown in Table 1 to produce a dry powder. The aim was to load 1 μg of final DPI (dry powder formulation of interferon-beta) into 1 mg of drying powder.

[Table 1]

| Parameter | Setting | Parameter | Setting |
|---|---|---|---|
| Feedstock Concentration | 2 ml/min | Drying Air Flow | 16-19 kg/h |
| Atomisation Pressure | 2.5 bar | Drying Air Pressure | 2.5 bar |
| Atomisation Flow | 15-18 L/min | Inlet Temperature | 100 - 120 °C |
| | | Outlet Temperature | 60 - 70 °C |

The particle size distribution of the dry powder composition was analyzed using a Malvern Mastersizer MS3000 device, and all powder products were stored under humidity conditions of 30% or less in a Safetech Powder Containment booth.

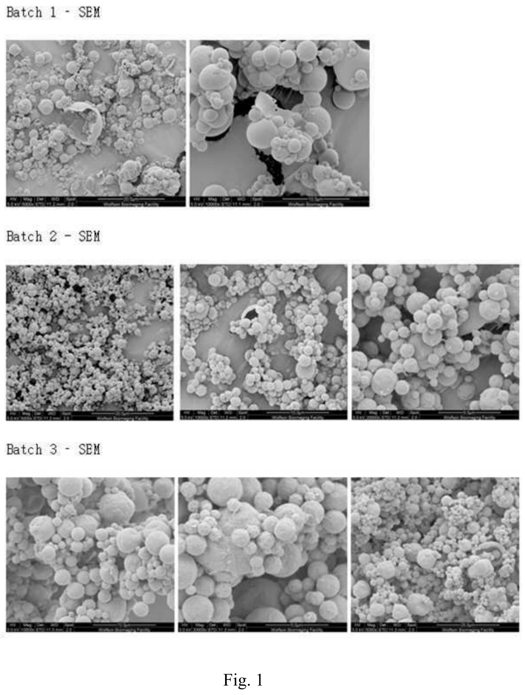

As a result, as shown in Table 2, the recovery rate of the dry powder formulation of interferon-beta increased in the composition containing 20% (w/v) leucine, and D90 (90% of particle size) was confirmed to be 6 μm or less. Furthermore, as shown in Figure 1, the particle morphology of each batch was confirmed using Scanning Electron Microscopy, and it was observed that spherical particles were uniformly formed.

[Table 2]

| | Leucine Content (%) | Yield (%) | Particle Size D10 (um) | Particle Size D50 (um) | Particle Size D90 (um) |
|---|---|---|---|---|---|
| Batch 1 (Original) | 0 | 26 | 1.1 | 3.1 | 7.1 |
| Batch 2 | 10 | 17 | 0.9 | 1.8 | 3.2 |
| Batch 3 | 20 | 74 | 1.1 | 2.5 | 5.4 |

**Example 2: Moisture Content Analysis of The Dry Powder Formulation of Interferon-Beta (Dry-Powder Inhalation (DPI))**

To measure the moisture content of the DPI(dry powder formulation of interferon-beta) manufactured in Example 1, the moisture content of the powder was measured using the Karl Fischer titration method.

All experimental conditions were conducted in facilities with humidity below 30%, and the analysis was performed using a Metrohm 851 Titrando Karl Fischer Coulometer. The analysis conditions are shown in Table 3.

[Table 3]

| Parameter | Setting |
|---|---|
| Nitrogen Flow | 50 mL/min (±10 mL/min) |
| Stirrer Speed | 8 |

(continued)

| Parameter | Setting |
|---|---|
| Drift | < 10 ug/min |
| Oven Temperature | 150 or 180 °C |
| Sample Weight | 50 mg ($\pm$5 mg) |
| Extraction Time | 120 seconds |

As a result, the moisture content of the dry powder formulation of interferon-beta was found to be 3.4%, which was measured to be at a suitable level for DPI formulations. Additionally, when the oven temperature was set to 150°C or 180°C for measurement, the moisture content of the powder was analyzed to be 3.46% and 3.41%, respectively, as shown in Table 4.

[Table 4]

| Oven Temperature | | 180 °C |
|---|---|---|
| **Sample Name** | **Weight** | **% of Water Content** |
| Repeat 1 | 0.04566 | 3.50 |
| Repeat 2 | 0.05267 | 3.43 |
| Repeat 3 | 0.05358 | 3.45 |
| **Average % of Water Content** | | **3.46** |
| **Oven Temperature** | | **150 °C** |
| **Sample Name** | **Weight** | **% of Water Content** |
| Repeat 1 | 0.05386 | 3.45 |
| Repeat 2 | 0.04768 | 3.39 |
| Repeat 3 | 0.04603 | 3.39 |
| **Average % of Water Content** | | **3.41** |

## Example 3: Recovery Rate Analysis of The Dry Powder Formulation of Interferon-Beta (Dry-Powder Inhalation (DPI))

To analyze the components of the dry powder formulation of interferon-beta manufactured in Example 1, the total protein amount was measured using a UV spectrophotometer, and the interferon-beta content was measured using SEC-HPLC.

Using the Evolution 260 Bio-UV Spectrophotometer, the dry powder formulation of interferon-beta was rehydrated and analyzed. For rehydration, the powder was dissolved in sterile water to prepare a concentration of 13.5-14.5 $\mu$g/mL, and the value obtained by subtracting the value of A320 nm wavelength from the value of A280 nm wavelength was measured.

Additionally, to measure the amount of interferon-beta contained in the rehydrated powder, the analysis was performed using SEC-HPLC under the conditions shown in Table 5, using the same rehydrated sample as that used for UV analysis.

[Table 5]

| | |
|---|---|
| **Mobile Phase** | 100 mM sodium phosphate, 150 mM sodium Chloride, pH 7.0 |
| **Sample Tray Temperature** | 4 $\pm$ 2 °C |
| **Injection Volume** | 50 $\mu$L |
| **Column** | TSKgel G2000SW XL 7.8 x 3000 mm, 5 $\mu$m |
| **Column Temperature** | 25 $\pm$ 5 °C |
| **Detector Wavelength** | 280 nm, 214 nm |

(continued)

| Run Time | 40 min |
|---|---|
| Flow Rate | 0.5 mL/min |

To confirm the actual amount of interferon-beta contained in the powder, the recovery rate was calculated using the values measured by UV and SEC-HPLC according to the following formula.

- UV protein concentration

$$\frac{Mean\ Absorbance * 1,000}{[(A280*1.78) * Sample\ path\ length]}$$

- % w/w concentration

$$\frac{[Protein\ concentration(ug/mL)/\ 1,000] * 100}{powder\ Weigh\ (mg)}$$

- % theoretical content

$$\frac{[\%\ w/w\ concentration] * 100}{\%\ Theoretical\ nominal\ content\ (\%\ w/w)}$$

As a result, as shown in Table 6, it was confirmed that 100% of the interferon-beta was recovered in the powder.

[Table 6]

| | Recovery (ug/mL) | | % w/w | | % of Theoretical |
|---|---|---|---|---|---|
| Active 1 | Theoretical | Actual | Theoretical | Actual | |
| Protein Content UV | 7.0 | 7.03 | 0.1 | 0.100 | 100 |
| SEC-HPLC Assay | | 6.78 | | 0.097 | 97 |
| Active 2 (10% leu) | | | | | |
| Protein Content UV | 13.7 | 16.1 | 0.1 | 0.118 | 118 |
| SEC-HPLC Assay | | 14.2 | | 0.1 | 100 |
| Active 3 (20% leu) | | | | | |
| Protein Content UV | 14.0 | 26.9 | 0.1 | 0.192 | 192 |
| SEC-HPLC Assay | | 14.8 | | 0.106 | 106 |

**Example 4: Activity Measurement of The Dry Powder Formulation of Interferon-Beta (Dry-Powder Inhalation (DPI))**

To measure the antiviral activity of the dry powder formulation of interferon-beta manufactured in Example 1, the powder was rehydrated, and ELISA and CPE analyses were performed. As a control group, a liquid composition of the same formulation that was not spray-dried was used.

To rehydrate the dry powder formulation of interferon-beta, 0.21 g of the dry powder was dissolved in 2.1 mL of sterile water to achieve an interferon-beta content of 0.1 mg/mL, thereby converting the powder into a liquid state. Using this solution, ELISA analysis was performed using a Toray ELISA kit, and antiviral activity was analyzed using a CPE assay.

For ELISA analysis, the liquid composition and the rehydrated dry powder formulation were diluted approximately 260,000-fold with ELISA-kit dilution buffer solution. The diluted samples were aliquoted into 96-well plates coated with anti-human interferon-beta antibody. Subsequently, an HRP-conjugates was added, and the mixture was reacted for 2 hours at 25°C while shaking at 400 rpm. After washing three times with 0.5% Tween-20 PBS, TMB solution was added and reacted again at 25°C for 30 minutes. The reaction was stopped with a stop solution, and absorbance was measured at 450 nm. The measured absorbance was substituted into a standard solution curve and converted to MIU/mL.

The converted MIU/mL was divided by interferon-beta mg/mL to calculate the specific activity (MIU/mg), and the reduction rate was measured by comparing it with the liquid composition before powdering.

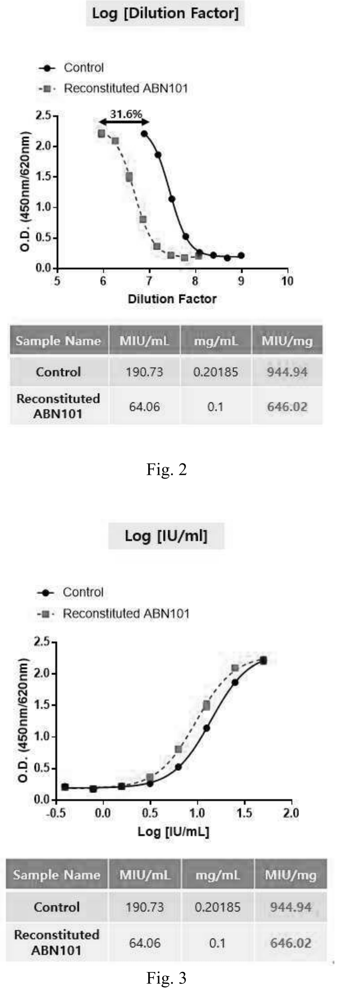

As a result, as shown in Figure 2, the specific activity of the rehydrated dry powder formulation (Reconstituted ABN101) was confirmed to have decreased by 31.6% compared to the liquid composition before powdering (Control).

For intracellular antiviral activity measurement, A549 cells were cultured in MEM media containing 2% heat-inactivated FBS, 100x sodium pyruvate, and 100x non-essential amino acids. The cells were seeded in 96-well plates at $2 \times 10^5$ cells/well, and the liquid composition or rehydrated powder composition was diluted to 50 IU/mL in the media and incubated at 37°C in a 5% $CO_2$ incubator for 22 hours. The liquid composition before powdering (Control) was diluted from 1/7,100,000, and the rehydrated dry powder (Reconstituted ABN101) was diluted from 1/900,000 for treatment. After 22 hours, the drug was removed, and EMCV stock was infected at approximately 1,000 TCID50/mL for 22 hours. Subsequently, EMCV was removed, and WST-8 solution was treated for 2 hours to measure cell viability (%) and determine antiviral activity. The calculation method for intracellular antiviral activity measurement is as follows.

CPE Assay (IU/mL)

= Standard activity (IU/mL) of $1^{st}$ row x $2^{(Nsam-Nstd)}$ x dilution fold number of $1^{st}$ row sample

*Ac: Absorbance mean value of cell control*

*Vc: Absorbance mean value of virus control*

$$A_{50}: (Ac+Vc)/2$$

$$N_{std}: n + (A_n-A_{50})/(A_n-A_{n+1})$$

$$N_{sam}: n + (A_n-A_{50})/(A_n-A_{n+1})$$

*N: Exponent of dilution times correspond to the lowest O.D. value among high O.D. value more than $A_{50}$*

**[Industrial Applicability]**

The dry powder formulation of IFN-β according to the present disclosure is a dry powder formulation of IFN-β having excellent morphological completeness and activity, and can effectively deliver IFN-β to the respiratory tract through inhalation. Accordingly, the formulation can be very usefully applied to the effective in-vivo delivery of IFN-β for uses including the treatment of respiratory diseases and respiratory viral infections, and thus has significant industrial applicability.

**Claims**

1. A dry powder formulation of interferon-beta prepared by spray drying a liquid composition, wherein the liquid composition comprises:

    (a) interferon beta or a variant thereof;
    (b) an acetate buffer at a concentration of 5 to 100 mM;
    (c) arginine at a concentration of 10 to 150 mM;
    (d) poloxamer 188 at a concentration of 0.1 to 10 mg/mL;
    (e) methionine at a concentration of 0.5 to 5 mM; and
    (f) 5 to 30% (w/v) of a hydrophobic amino acid.

2. The dry powder formulation according to claim 1, wherein the interferon beta variant is a variant of human interferon beta in which the 27th amino acid, arginine, is substituted with threonine.

3. The dry powder formulation according to claim 1, wherein the interferon beta variant comprises an N-linked glycan at the 25th amino acid residue, asparagine, of human interferon beta.

4. The dry powder formulation according to claim 1, wherein the acetate buffer has a pH range of 3.6 to 4.4.

5. The dry powder formulation according to claim 1, wherein the interferon beta or a variant thereof is comprised at a concentration of 0.01 to 1% (w/v) in the liquid composition.

6. The dry powder formulation according to claim 1, wherein the hydrophobic amino acid is selected from the group consisting of tryptophan, tyrosine, leucine, and phenylalanine.

7. The dry powder formulation according to claim 1, wherein the hydrophobic amino acid is included at a concentration of 15 to 25% (w/v) in the liquid composition.

8. The dry powder formulation according to claim 1, wherein the formulation has a particle size of 0.1 $\mu$m to 10 $\mu$m in 90% (w/w) of the dry powder.

9. The dry powder formulation according to claim 1, wherein the formulation is for use in inhalation administration.

10. An inhalation capsule filled with the dry powder formulation according to any one of claims 1 to 9.

11. A pharmaceutical composition comprising the dry powder formulation according to any one of claims 1 to 9 for use in preventing or treating a disease selected from the group consisting of multiple sclerosis, cancer, autoimmune diseases, viral infectious diseases, HIV infectious diseases, hepatitis C, and rheumatoid arthritis.

12. A method for preparing a dry powder formulation of interferon-beta, comprising spray drying a liquid composition at an inlet temperature of 90 to 130°C and an outlet temperature of 40 to 80°C, wherein the liquid composition comprises:

   (a) interferon beta or a variant thereof;
   (b) an acetate buffer at a concentration of 5 to 100 mM;
   (c) arginine at a concentration of 10 to 150 mM;
   (d) poloxamer 188 at a concentration of 0.1 to 10 mg/mL;
   (e) methionine at a concentration of 0.5 to 5 mM; and
   (f) 5 to 30% (w/v) of a hydrophobic amino acid.

13. Use of the dry powder formulation according to any one of claims 1 to 9 for preparing a composition for treating a disease selected from the group consisting of multiple sclerosis, cancer, autoimmune diseases, viral infectious diseases, HIV infectious diseases, hepatitis C, and rheumatoid arthritis.

14. A method for treating a disease comprising administering an effective amount of a composition,

   wherein the disease is selected from the group consisting of multiple sclerosis, cancer, autoimmune diseases, viral infectious diseases, HIV infectious diseases, hepatitis C, and rheumatoid arthritis; and
   wherein the composition comprises administering an effective amount of a composition comprising the dry powder formulation according to any one of claims 1 to 9 as an active ingredient to a subject in need thereof.

Batch 1 - SEM

Batch 2 - SEM

Batch 3 - SEM

Fig. 1

## Log [Dilution Factor]

| Sample Name | MIU/mL | mg/mL | MIU/mg |
|---|---|---|---|
| Control | 190.73 | 0.20185 | 944.94 |
| Reconstituted ABN101 | 64.06 | 0.1 | 646.02 |

Fig. 2

## Log [IU/ml]

| Sample Name | MIU/mL | mg/mL | MIU/mg |
|---|---|---|---|
| Control | 190.73 | 0.20185 | 944.94 |
| Reconstituted ABN101 | 64.06 | 0.1 | 646.02 |

Fig. 3

## INTERNATIONAL SEARCH REPORT

International application No.

**PCT/KR2024/001270**

### A. CLASSIFICATION OF SUBJECT MATTER

**A61K 9/14**(2006.01)i; **A61K 47/12**(2006.01)i; **A61K 47/18**(2006.01)i; **A61K 47/34**(2006.01)i; **A61K 47/22**(2006.01)i; **A61K 9/00**(2006.01)i; **A61K 38/21**(2006.01)i; **A61P 25/28**(2006.01)i; **A61P 37/00**(2006.01)i; **A61P 35/00**(2006.01)i

According to International Patent Classification (IPC) or to both national classification and IPC

### B. FIELDS SEARCHED

Minimum documentation searched (classification system followed by classification symbols)

A61K 9/14(2006.01); A61K 38/21(2006.01); A61K 47/10(2006.01); A61K 47/18(2006.01); A61K 9/08(2006.01); C07K 14/565(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인터페론 베타(Interferon beta), 아세트산(acetic acid), 아르기닌 (arginine), 폴록사머 188(poloxamer 188), 메티오닌(methionine), 소수성 아미노산(Hydrophobic amino acid), 분무건조 (spray drying), 분말(powder)

### C. DOCUMENTS CONSIDERED TO BE RELEVANT

| Category* | Citation of document, with indication, where appropriate, of the relevant passages | Relevant to claim No. |
|---|---|---|
| Y | KR 10-2016-0120239 A (ABION INC.) 17 October 2016 (2016-10-17)<br>See claims 1-4 and 10-12, and paragraph [0027]. | 1-13 |
| Y | US 2003-0035778 A1 (PLATZ, Robert et al.) 20 February 2003 (2003-02-20)<br>See claims 1 and 10, and paragraphs [0033], [0037], [0042], [0046]-[0047] and [0051]. | 1-13 |
| A | KR 10-2006-0039132 A (SAMSUNG FINE CHEMICALS CO., LTD.) 08 May 2006 (2006-05-08)<br>See claims 1-10 and 14-16. | 1-13 |
| A | KR 10-2006-0011976 A (ARES TRADING S.A.) 06 February 2006 (2006-02-06)<br>See claims 1-9 and 17-22. | 1-13 |
| A | KR 10-2003-0097825 A (CHIRON CORPORATION) 31 December 2003 (2003-12-31)<br>See claims 1 and 13. | 1-13 |

☐ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 May 2024** | **02 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<div align="center">

**INTERNATIONAL SEARCH REPORT**

</div>

| | International application No. |
|---|---|
| | **PCT/KR2024/001270** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **14**
   because they relate to subject matter not required to be searched by this Authority, namely:

   Claim 14 pertains to a method for treatment of the human body by means of a composition (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| | International application No. |
|---|---|
| | **PCT/KR2024/001270** |

| Patent document cited in search report | | | Publication date (day/month/year) | Patent family member(s) | | | Publication date (day/month/year) |
|---|---|---|---|---|---|---|---|
| KR | 10-2016-0120239 | A | 17 October 2016 | KR | 10-1781945 | B1 | 26 September 2017 |
| | | | | WO | 2016-163764 | A2 | 13 October 2016 |
| | | | | WO | 2016-163764 | A3 | 01 December 2016 |
| US | 2003-0035778 | A1 | 20 February 2003 | AP | 9801369 | A0 | 31 December 1998 |
| | | | | AP | 987 | A | 01 August 2001 |
| | | | | AR | 013854 | A1 | 31 January 2001 |
| | | | | AR | 042921 | A2 | 06 July 2005 |
| | | | | AT | 189124 | T | 15 February 2000 |
| | | | | AT | 416755 | T | 15 December 2008 |
| | | | | AT | E299892 | T1 | 15 August 2005 |
| | | | | AU | 1992-23091 | A | 11 February 1993 |
| | | | | AU | 1997-31190 | A | 26 November 1997 |
| | | | | AU | 1999-23695 | A | 08 July 1999 |
| | | | | BG | 102875 | A | 31 May 1999 |
| | | | | BG | 64113 | B1 | 30 January 2004 |
| | | | | BR | 9307141 | A | 30 March 1999 |
| | | | | BR | 9507023 | A | 23 September 1997 |
| | | | | CA | 2112674 | A1 | 21 January 1993 |
| | | | | CA | 2183577 | A1 | 14 September 1995 |
| | | | | CA | 2253393 | A1 | 13 November 1997 |
| | | | | CA | 2555600 | A1 | 28 March 1996 |
| | | | | CN | 1494951 | A | 12 May 2004 |
| | | | | CO | 5011099 | A1 | 28 February 2001 |
| | | | | CZ | 260096 | A3 | 16 April 1997 |
| | | | | CZ | 359998 | A3 | 17 March 1999 |
| | | | | CZ | 72397 | A3 | 12 November 1997 |
| | | | | CZ | 80395 | A3 | 15 November 1995 |
| | | | | DE | 69230613 | T2 | 28 December 2000 |
| | | | | DE | 69723854 | T2 | 22 April 2004 |
| | | | | DK | 0592540 | T3 | 26 June 2000 |
| | | | | DK | 0941067 | T3 | 17 November 2003 |
| | | | | EA | 199900454 | A1 | 29 December 1999 |
| | | | | EE | 9800376 | A | 15 April 1999 |
| | | | | EP | 0592540 | A1 | 20 April 1994 |
| | | | | EP | 0748213 | B1 | 14 April 2004 |
| | | | | EP | 1342469 | A2 | 10 September 2003 |
| | | | | EP | 2036541 | A1 | 18 March 2009 |
| | | | | ES | 2141108 | T3 | 16 March 2000 |
| | | | | ES | 2217309 | T3 | 01 November 2004 |
| | | | | FI | 951465 | A0 | 28 March 1995 |
| | | | | FI | 963468 | A0 | 04 September 1996 |
| | | | | FI | 971173 | A0 | 20 March 1997 |
| | | | | GR | 3032973 | T3 | 31 July 2000 |
| | | | | HK | 1020319 | A1 | 14 April 2000 |
| | | | | HK | 1024618 | A1 | 20 October 2000 |
| | | | | HU | 0001108 | A2 | 28 August 2000 |
| | | | | HU | T77691 | A | 28 July 1998 |
| | | | | IL | 112618 | A0 | 29 June 1995 |
| | | | | IL | 130476 | A0 | 01 June 2000 |
| | | | | IS | 4879 | A | 26 October 1998 |

Form PCT/ISA/210 (patent family annex) (July 2022)

## INTERNATIONAL SEARCH REPORT
**Information on patent family members**

International application No.

**PCT/KR2024/001270**

| Patent document cited in search report | | | Publication date (day/month/year) | Patent family member(s) | | | Publication date (day/month/year) |
|---|---|---|---|---|---|---|---|
| | | | | JP | 2000-510471 | A | 15 August 2000 |
| | | | | JP | 2009-191071 | A | 27 August 2009 |
| | | | | KR | 10-1994-7001282 | A | 28 May 1994 |
| | | | | KR | 10-2000-0010807 | A | 25 February 2000 |
| | | | | LT | 4553 | B | 25 October 1999 |
| | | | | LT | 98157 | A | 25 May 1999 |
| | | | | LV | 12231 | A | 20 March 1999 |
| | | | | LV | 12231 | B | 20 October 1999 |
| | | | | MX | 9603936 | A | 31 May 1997 |
| | | | | MX | 9707855 | A | 28 February 1998 |
| | | | | MY | 113727 | A | 31 May 2002 |
| | | | | MY | 124282 | A | 30 June 2006 |
| | | | | NO | 20063519 | A | 20 May 1997 |
| | | | | NO | 20080655 | A | 20 May 1997 |
| | | | | NO | 20080655 | L | 20 May 1997 |
| | | | | NO | 951192 | A | 28 March 1995 |
| | | | | NO | 971316 | A | 20 May 1997 |
| | | | | NZ | 257212 | A | 26 November 1996 |
| | | | | NZ | 299241 | A | 24 November 2000 |
| | | | | OA | 10914 | A | 26 October 2001 |
| | | | | PE | 16397 | A1 | 10 May 1997 |
| | | | | PE | 48995 | A1 | 13 January 1996 |
| | | | | PL | 316199 | A1 | 23 December 1996 |
| | | | | PL | 319505 | A1 | 18 August 1997 |
| | | | | PT | 748213 | E | 31 August 2004 |
| | | | | PT | 941067 | E | 31 December 2003 |
| | | | | RO | 118523 | B1 | 30 June 2003 |
| | | | | RU | 95112536 | A | 20 December 1996 |
| | | | | SI | 0941067 | T1 | 29 February 2004 |
| | | | | SI | 9720031 | A | 28 February 1999 |
| | | | | SK | 152598 | A3 | 13 April 1999 |
| | | | | SK | 285400 | B6 | 04 January 2007 |
| | | | | TR | 9802247 | T2 | 21 November 2001 |
| | | | | TW | 504389 | A | 01 October 2002 |
| | | | | UA | 65538 | C2 | 15 April 2004 |
| | | | | US | 5458135 | A | 17 October 1995 |
| | | | | US | 5607915 | A | 04 March 1997 |
| | | | | US | 5775320 | A | 07 July 1998 |
| | | | | US | 5997848 | A | 07 December 1999 |
| | | | | US | 6019968 | A | 01 February 2000 |
| | | | | US | 6051256 | A | 18 April 2000 |
| | | | | US | 6165463 | A | 26 December 2000 |
| KR | 10-2006-0039132 | A | 08 May 2006 | AT | 509951 | T | 15 June 2011 |
| | | | | AT | E509951 | T1 | 15 June 2011 |
| | | | | BR | PI0517932 | A | 21 October 2008 |
| | | | | CN | 101111519 | A | 23 January 2008 |
| | | | | DK | 1809661 | T3 | 05 September 2011 |
| | | | | EP | 1809661 | A1 | 25 July 2007 |
| | | | | ES | 2369088 | T3 | 25 November 2011 |
| | | | | JP | 2008-518631 | A | 05 June 2008 |

Form PCT/ISA/210 (patent family annex) (July 2022)

## INTERNATIONAL SEARCH REPORT
### Information on patent family members

| International application No. |
| --- |
| **PCT/KR2024/001270** |

| Patent document cited in search report | | | Publication date (day/month/year) | Patent family member(s) | | | Publication date (day/month/year) |
| --- | --- | --- | --- | --- | --- | --- | --- |
| | | | | PT | 1809661 | E | 05 September 2011 |
| | | | | US | 2010-0003721 | A1 | 07 January 2010 |
| | | | | WO | 2006-049423 | A1 | 11 May 2006 |
| KR | 10-2006-0011976 | A | 06 February 2006 | AR | 044147 | A1 | 24 August 2005 |
| | | | | AU | 2004-233603 | A1 | 11 November 2004 |
| | | | | AU | 2009-201261 | A1 | 23 April 2009 |
| | | | | BR | PI0410488 | A | 13 June 2006 |
| | | | | BR | PI0410488 | B1 | 22 June 2021 |
| | | | | CA | 2521560 | A1 | 11 November 2004 |
| | | | | CN | 1816347 | A | 09 August 2006 |
| | | | | CY | 1114273 | T1 | 05 October 2016 |
| | | | | DK | 1617861 | T3 | 29 July 2013 |
| | | | | EA | 009995 | B1 | 30 June 2008 |
| | | | | EA | 200501699 | A1 | 30 June 2006 |
| | | | | EP | 1617861 | A2 | 25 January 2006 |
| | | | | ES | 2417061 | T3 | 05 August 2013 |
| | | | | HK | 1088847 | A1 | 17 November 2006 |
| | | | | HR | P20130512 | T1 | 31 July 2013 |
| | | | | IL | 171709 | A | 12 March 2006 |
| | | | | JP | 2006-525280 | A | 09 November 2006 |
| | | | | JP | 2011-225599 | A | 10 November 2011 |
| | | | | ME | P60408 | A | 10 May 2011 |
| | | | | MX | PA05011718 | A | 23 January 2006 |
| | | | | MY | 151121 | A | 30 April 2014 |
| | | | | NO | 20055666 | A | 30 November 2005 |
| | | | | NO | 335674 | B1 | 19 January 2015 |
| | | | | NZ | 542912 | A | 30 April 2008 |
| | | | | PT | 1617861 | E | 28 August 2013 |
| | | | | RS | 20050821 | A | 31 December 2007 |
| | | | | SG | 159389 | A1 | 30 March 2010 |
| | | | | SI | 1617861 | T1 | 30 September 2013 |
| | | | | TW | 200503754 | A | 01 February 2005 |
| | | | | UA | 94032 | C2 | 11 April 2011 |
| | | | | US | 2007-0059285 | A1 | 15 March 2007 |
| | | | | WO | 2004-096263 | A2 | 11 November 2004 |
| | | | | ZA | 200508124 | A | 25 February 2009 |
| | | | | ZA | 200708484 | A | 29 October 2008 |
| KR | 10-2003-0097825 | A | 31 December 2003 | AT | 545430 | T | 15 March 2012 |
| | | | | AU | 2002-241769 | C1 | 01 June 2006 |
| | | | | BG | 108322 | A | 31 August 2005 |
| | | | | CA | 2442854 | A1 | 17 October 2002 |
| | | | | CA | 2762966 | A1 | 17 October 2002 |
| | | | | CN | 1511053 | A | 07 July 2004 |
| | | | | CY | 1113609 | T1 | 05 August 2015 |
| | | | | CZ | 20032735 | A3 | 16 March 2005 |
| | | | | DK | 1381432 | T3 | 12 March 2012 |
| | | | | EP | 1381432 | A2 | 21 January 2004 |
| | | | | EP | 2283896 | A2 | 16 February 2011 |
| | | | | EP | 2283897 | A2 | 16 February 2011 |
| | | | | ES | 2367761 | T1 | 08 November 2011 |

Form PCT/ISA/210 (patent family annex) (July 2022)

## INTERNATIONAL SEARCH REPORT
### Information on patent family members

International application No.

**PCT/KR2024/001270**

| Patent document cited in search report | Publication date (day/month/year) | Patent family member(s) | | Publication date (day/month/year) |
|---|---|---|---|---|
| | | ES | 2453623 T3 | 08 April 2014 |
| | | HU | 0303732 A2 | 28 September 2007 |
| | | IL | 157963 A0 | 28 March 2004 |
| | | JP | 2004-529917 A | 30 September 2004 |
| | | JP | 2015-044882 A | 12 March 2015 |
| | | LU | 92060 I2 | 15 October 2012 |
| | | NO | 20034492 A | 25 November 2003 |
| | | NO | 330259 B1 | 14 March 2011 |
| | | PL | 213297 B1 | 28 February 2013 |
| | | PL | 366790 A1 | 07 February 2005 |
| | | PT | 1381432 E | 03 April 2012 |
| | | PT | 2283896 E | 16 December 2014 |
| | | US | 2002-0172661 A1 | 21 November 2002 |
| | | US | 2011-0104116 A1 | 05 May 2011 |
| | | WO | 02-080976 A2 | 17 October 2002 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230010191
- WO 9116038 A